# EUROPEAN PATENT APPLICATION

(11) **EP 3 581 620 A1**
(43) Date of publication of application: **18.12.2019**
(21) Application number: 18764540.3
(22) Date of filing: 02.03.2018
(51) Int. Cl.: C08L 71/02, A61L 31/14, B01J 13/00, C08J 3/075, C08K 3/32, C08K 3/34, C08K 5/092

(54) **TEMPERATURE-RESPONSIVE HYDROGEL AND METHOD FOR PRODUCING SAME**

(30) Priority: 09.03.2017 JP 2017045190
(71) Applicant: Nissan Chemical Corporation, Tokyo 103-6119 (JP); Nihon University, Tokyo 102-8275 (JP)
(72) Inventor: KUDO, Yoshihiro, Funabashi-shi Chiba 274-0052 (JP); HARAGUCHI, Kazutoshi, Tokyo 102-8275 (JP)
(74) Representative: Hoffmann Eitle
(86) International application number: PCT/JP2018/008088
(87) International publication number: WO 2018/164003

(57) **Abstract**

There is provided a temperature-responsive hydrogel exhibiting a high viscosity at a high temperature and a low viscosity at a low temperature obtained by means of a simple method (mixing).

It is found that a temperature-responsive hydrogel exhibiting a high viscosity at a high temperature and a low viscosity at a low temperature and a high ratio of the viscosity at a high temperature to the viscosity at a low temperature can be produced by mixing of an aqueous solution containing a polyalkylene glycol (A) having a weight average molecular weight of 20,000 to 10,000,000 with an aqueous dispersion containing a silicate salt (B) so as to achieve a specific compositional range of the components in the hydrogel, specifically, at a mass concentration of A of 0.01 to 1.5% by mass, preferably at a mass concentration of A of 0.1 to 0.4% by mass and at a mass concentration of B of 0.5 to 10% by mass, preferably at a mass concentration of B of 1 to 5% by mass, with the mass ratio R of A to B (R=(mass of A) / (mass of B)) being within the range of 0.01 to 0.5. The present invention has been accomplished on the basis of this finding.

## Description

### TECHNICAL FIELD

The present invention relates to a temperature-responsive hydrogel and a method for producing the hydrogel. More particularly, the present invention relates to a temperature-responsive hydrogel containing a polyalkylene glycol and a silicate salt, and a method for producing the hydrogel.

### BACKGROUND ART

Temperature-responsive hydrogel has been developed as one of biocompatible stimulus-responsive materials for applications, for example, in the medical field, including drug delivery systems (DDS), regenerative medicine, and microactuators.

An aqueous dispersion containing polyethylene glycol (PEG) and a layered clay mineral (i.e., water-swellable silicate salt particles) has been reported to have temperature dependence (15 to 40°C) of viscoelasticity (Non-Patent Document 1). According to this document, the results of measurement of the viscosities of aqueous dispersions containing PEG having an average molecular weight of 10,000 at a fixed concentration of 2% by mass and containing a clay mineral (LAPONITE RD) at different concentrations (1 to 4% by mass) indicated that a LAPONITE RD concentration of 1 to 3% by mass led to a common change in viscosity (i.e., a decrease in zero shear viscosity with an increase in temperature), whereas a LAPONITE RD concentration of 4% by mass led to a temperature response such that the zero shear viscosity was higher at 35°C than at room temperature (the zero shear viscosity slightly decreased again at 40°C). Thus, an uncommon temperature dependence of viscosity (high viscosity at high temperature) was observed in such a limited composition having a relatively high concentration (2% by mass) of PEG and a further high concentration (4% by mass) of the clay mineral. However, an aqueous dispersion for practical use is required to exhibit a temperature response such that the viscosity changes greatly even at lower concentrations of components. No studies have been conducted on the temperature dependence of viscosity in the case of use of PEG having an average molecular weight as high as several hundreds of thousands or more.

### Prior Art Documents

### Non-Patent Documents

Non-Patent Document 1: JOURNAL of Physical Chemistry B (2012), 116 (1), 48-54

### SUMMARY OF THE INVENTION

### Problems to be Solved by the Invention

In view of the foregoing, an object of the present invention is to provide a temperature-responsive hydrogel exhibiting a high viscosity at a high temperature and a low viscosity at a low temperature; i.e., a viscosity profile such that the viscosity at a high temperature is higher than that at a low temperature.

### Means for Solving the Problems

The present inventors have conducted extensive studies for solving the aforementioned problems, and as a result have found that a temperature-responsive hydrogel exhibiting a high ratio of the viscosity at a high temperature to the viscosity at a low temperature can be produced by mixing of an aqueous solution containing a polyalkylene glycol having a weight average molecular weight of 20,000 to 10,000,000 with an aqueous dispersion containing a silicate salt so as to achieve a specific compositional range of the components in the hydrogel. The present invention has been accomplished on the basis of this finding.

Accordingly,
a first aspect of the present invention is a temperature-responsive hydrogel comprising a polyalkylene glycol (A) and a silicate salt (B), wherein the concentration by mass of the polyalkylene glycol (A) is 0.01 to 1.5% by mass in the hydrogel;
a second aspect of the present invention is the temperature-responsive hydrogel according to the first aspect, wherein the temperature-responsive hydrogel further comprises a dispersant (C) for the silicate salt;
a third aspect of the present invention is the temperature-responsive hydrogel according to the first or second aspect, wherein the polyalkylene glycol (A) is polyethylene glycol or polypropylene glycol having a weight average molecular weight of 20,000 to 10,000,000;
a fourth aspect of the present invention is the temperature-responsive hydrogel according to any one of the first to third aspects, wherein the concentration by mass of the silicate salt (B) is 0.5 to 10% by mass in the hydrogel;
a fifth aspect of the present invention is the temperature-responsive hydrogel according to any one of the first to fourth aspects, wherein the silicate salt (B) is water-swellable silicate salt particles;
a sixth aspect of the present invention is the temperature-responsive hydrogel according to the fifth aspect, wherein the water-swellable silicate salt particles are particles of a water-swellable silicate salt selected from the group consisting of smectite, bentonite, vermiculite, and mica;
a seventh aspect of the present invention is the temperature-responsive hydrogel according to any one of the first to sixth aspects, wherein the mass ratio R of the polyalkylene glycol (A) to the silicate salt (B) (R = [the mass of A]/[the mass of B]) is 0.01 to 0.5;
an eighth aspect of the present invention is the temperature-responsive hydrogel according to any one of the second to seventh aspects, wherein the dispersant (C) is at least one dispersant selected from the group consisting of sodium pyrophosphate, citric acid, and polyphosphoric acid;
a ninth aspect of the present invention is the temperature-responsive hydrogel according to any one of the first to eighth aspects, wherein the viscosity of the hydrogel at a temperature of 50 to 100°C is twice or more the viscosity of the hydrogel at a temperature of 0 to 25°C;
a tenth aspect of the present invention is the temperature-responsive hydrogel according to any one of the first to eighth aspects, wherein the hydrogel exhibits a viscosity of 600 to 10,000 mPa·s at a temperature of 50 to 100°C and a viscosity of 100 to 580 mPa·s at a temperature of 0 to 25°C;
an eleventh aspect of the present invention is the temperature-responsive hydrogel according to any one of the first to eighth aspects, wherein the hydrogel exhibits a viscosity of 3,000 to 10,000 mPa·s at a temperature of 50 to 100°C and a viscosity of 500 to 2,980 mPa·s at a temperature of 0 to 25°C;
a twelfth aspect of the present invention is a method for producing a temperature-responsive hydrogel, the method comprising a step of mixing an aqueous solution containing a polyalkylene glycol (A) at a concentration by mass of 0.02 to 3.0% by mass with an aqueous dispersion containing a silicate salt (B);
a thirteenth aspect of the present invention is the method for producing a temperature-responsive hydrogel according to the eleventh aspect, wherein one or more water-soluble organic solvents (D) are contained in either or both of the aqueous solution and the aqueous dispersion before mixing of the aqueous solution and the aqueous dispersion; and
a fourteenth aspect of the present invention is the temperature-responsive hydrogel according to any one of the ninth to eleventh aspects, wherein the temperature-responsive hydrogel exhibits at least one reproducible change in viscosity of the hydrogel with respect to a change in temperature.

### Effects of the Invention

As described above, the present invention can produce a temperature-responsive hydrogel exhibiting a high viscosity at a high temperature and a low viscosity at a low temperature (i.e., an increase in viscosity with an increase in temperature and a decrease in viscosity with a decrease in temperature) and exhibiting a high ratio of the viscosity at a high temperature to the viscosity at a low temperature by mixing of an aqueous solution containing a polyalkylene glycol having a weight average molecular weight of 20,000 to 10,000,000 with an aqueous dispersion containing a silicate salt so as to achieve a specific compositional range of the components in the hydrogel.

### MODES FOR CARRYING OUT THE INVENTION

The present invention relates to a temperature-responsive hydrogel containing a specific polyalkylene glycol (A) and a silicate salt (B), which are components essential for gelation, and water.

The temperature-responsive hydrogel of the present invention may optionally contain, besides the aforementioned components, a dispersant (C) (e.g., a dispersant for the silicate salt) or an organic solvent (D) having compatibility with water, so long as such a component does not impair the intended effects of the present invention or enhances the effects of the invention.

### <Component (A): Polyalkylene Glycol>

The component (A) according to the present invention is a polyalkylene glycol. The polyalkylene glycol is, for example, polyethylene glycol or polypropylene glycol. The weight average molecular weight of the polyalkylene glycol (A) can be measured by gel permeation chromatography (GPC) in terms of polystyrene. The weight average molecular weight is, for example, 10,000 to 10,000,000, for example, 20,000 to 10,000,000, preferably 50,000 to 5,000,000, more preferably 100,000 to 2,000,000, most preferably 300,000 to 1,000,000.

### <Component (B): Silicate Salt>

The component (B) according to the present invention is a silicate salt and is preferably water-swellable silicate salt particles.

Examples of the silicate salt (B) include smectite, bentonite, vermiculite, and mica. The silicate salt (B) preferably forms a colloid with water or a water-containing solvent serving as a dispersion medium. The term "smectite" is a name of a group including montmorillonite, beidellite, nontronite, saponite, hectorite, and stevensite.

Primary particles of the silicate salt are in a disc-like, plate-like, spherical, particulate, cubic, acicular, rod-like, or amorphous form, for example. The silicate salt is preferably in the form of disk-like or plate-like particles having a diameter of 5 nm to 1,000 nm.

Preferred specific examples of the silicate salt include layered silicate salts. Examples of readily available commercial products include LAPONITE XLG (synthetic hectorite), LAPONITE XLS (synthetic hectorite containing sodium pyrophosphate as a dispersant), LAPONITE XL21 (sodium magnesium fluorosilicate), LAPONITE RD (synthetic hectorite), LAPONITE RDS (synthetic hectorite containing an inorganic polyphosphate salt as a dispersant), LAPONITE S482 (synthetic hectorite containing a dispersant), and LAPONITE EP (organic modified hectorite) manufactured by BYK Additives; KUNIPIA (registered trademark of Kunimine Industries Co., Ltd., montmorillonite), SUMECTON (registered trademark of Kunimine Industries Co., Ltd.) SA (synthetic saponite), SUMECTON ST (synthetic stevensite), SUMECTON SWN (synthetic hectorite), and SUMECTON SWF (fluorosynthetic hectorite) manufactured by Kunimine Industries Co., Ltd.; and BEN-GEL (registered trademark of HOJUN Co., Ltd., a purified product of natural bentonite) manufactured by HOJUN Co., Ltd.

### <Component (C): Dispersant>

The component (C) according to the present invention is used for uniformly dispersing the components of the gel or increasing a change in viscosity of the gel with temperature. Examples of the component (C) include sodium pyrophosphate, citric acid, and polyphosphoric acid. Of these, sodium pyrophosphate is preferred.

### <Component (D): Water-Soluble Organic Solvent>

The component (D) according to the present invention is used for uniformly dispersing the components of the gel or maintaining the flexibility of the gel after being dried. Examples of the component (D) include methanol, ethanol, propanol, isopropyl alcohol, acetone, glycerin, diglycerin, ethylene glycol, and 1,3-butylene glycol. Of these, glycerin, diglycerin, ethylene glycol, and 1,3-butylene glycol are preferred.

The concentrations of the components contained in the temperature-responsive hydrogel of the present invention can be appropriately adjusted, so long as the effects of the present invention are not impaired. For example, the concentration of the polyalkylene glycol (A) is 0.01 to 1.5% by mass, preferably 0.05 to 0.5% by mass, more preferably 0.1 to 0.4% by mass, the concentration of the silicate salt (B) is 0.5 to 10% by mass, preferably 1 to 5% by mass, the concentration of the dispersant (C) is 0.1 to 10% by mass, and the concentration of the water-soluble organic solvent (D) is 1 to 50% by mass, relative to the entire mass of the temperature-responsive hydrogel.

A polyalkylene glycol (A) concentration of less than 0.01% by mass may result in failure to produce a gel, whereas a polyalkylene glycol (A) concentration exceeding 1.5% by mass may result in failure to produce a temperature-responsive hydrogel exhibiting a high viscosity at a high temperature and a low viscosity at a low temperature.

When the concentration of the silicate salt (B) is adjusted to fall within the aforementioned range, a homogeneous temperature-responsive hydrogel exhibiting a high viscosity at a high temperature and a low viscosity at a low temperature can be readily produced without causing, for example, precipitation.

The mass ratio R of the polyalkylene glycol (A) to the silicate salt (B) (R = [the mass of A]/[the mass of B]) can be appropriately adjusted in the temperature-responsive hydrogel of the present invention, so long as the effects of the present invention are not impaired. The mass ratio R is, for example, 0.01 to 0.5, preferably 0.02 to 0.3, more preferably 0.03 to 0.2.

When the mass ratio R of the polyalkylene glycol (A) to the silicate salt (B) is adjusted to fall within the aforementioned range, the resultant temperature-responsive hydrogel exhibits a high ratio of the viscosity at a high temperature to the viscosity at a low temperature.

The temperature-responsive hydrogel of the present invention exhibits a high viscosity at a high temperature and a low viscosity at a low temperature; i.e., an increase in viscosity with an increase in temperature (a decrease in viscosity with a decrease in temperature), and also exhibits a high ratio of the viscosity at a high temperature to the viscosity at a low temperature.

The term "temperature" as used herein refers to Celsius degree (°C) in the atmosphere.

No particular limitation is imposed on the viscosity measuring method, and the viscosity is measured with, for example, a rotational viscometer or a tuning-fork vibration viscometer.

As used herein, the term "high temperature" refers to the higher one of two temperatures, whereas the term "low temperature" refers to the lower one of the two temperatures. The temperature difference between high and low temperatures falls within a temperature range wherein the temperature-responsive hydrogel exhibits its gel characteristics. For example, the temperature difference is 10°C or more, 20°C or more, 25°C or more, 30°C or more, 40°C or more, 50°C or more, 70°C or more, or 80°C or more. These temperatures are not necessarily limited to specific temperatures, but are, for example, set at a temperature higher than room temperature (e.g., 25°C) and a temperature lower than room temperature. Generally, the high temperature is set at a temperature higher than room temperature; for example, room temperature to 200°C, 50 to 100°C, 50 to 80°C, or 50 to 70°C, and the low temperature is set at a temperature lower than room temperature; for example, -5°C to room temperature, 0°C to room temperature, or 0°C to 10°C.

As used herein, the term "high viscosity" refers to the higher one of two viscosities measured at the above-set high and low temperatures (temperature difference), whereas the term "low viscosity" refers to the lower one of the two viscosities measured at the aforementioned two temperatures. The temperature-responsive hydrogel of the present invention exhibits a high ratio of the viscosity at a high temperature to the viscosity at a low temperature. This refers to the case where, for example, the ratio of the viscosity at the above-set high temperature to the viscosity at the above-set low temperature is more than 1; for example, 1.2 or more, 1.5 or more, 2 or more, 3 or more, 4 or more, 5 or more, or 10 or more. Preferably, the expression "high ratio of the viscosity at a high temperature to the viscosity at a low temperature" refers to the case where the viscosity ratio is 2 or more. These viscosities are not necessarily limited to specific viscosities. Generally, the combination of a high viscosity and a low viscosity is, for example, 300 to 2,000 mPa·s and 100 to 280 mPa·s, 600 to 10,000 mPa·s and 100 to 580 mPa·s, 600 to 5,000 mPa·s and 200 to 580 mPa·s, 600 to 5,000 mPa·s and 100 to 580 mPa·s, 1,000 to 7,000 mPa·s and 300 to 980 mPa·s, 1,000 to 2,500 mPa·s and 100 to 500 mPa·s, 1,000 to 5,000 mPa·s and 100 to 500 mPa·s, 2,000 to 10,000 mPa·s and 500 to 1,980 mPa·s, 3,000 to 10,000 mPa·s and 500 to 2,980 mPa·s, or 3,000 to 10,000 mPa·s and 1,000 to 2,980 mPa·s.

In one specific embodiment, the temperature-responsive hydrogel preferably exhibits a temperature response such that the viscosity at a temperature of 50 to 80°C is twice or more the viscosity at a temperature of 0 to 25°C.

Preferably, when the temperature of the temperature-responsive hydrogel is increased by 10°C, 20°C, 25°C, 30°C, 40°C, 50°C, 70°C, or 80°C within a temperature range wherein the temperature-responsive hydrogel exhibits its gel characteristics; for example, a temperature range of -20°C to 120°C, -5°C to 105°C, 0°C to 100°C, or 0°C to 90°C, the viscosity after the temperature increase is twice or more the viscosity before the temperature increase.

In one specific embodiment, the temperature-responsive hydrogel exhibits a high viscosity (600 to 10,000 mPa·s) at a high temperature (50 to 100°C) and a low viscosity (100 to 580 mPa·s) at a low temperature (0 to 25°C).

In one specific embodiment, the temperature-responsive hydrogel exhibits a high viscosity (600 to 5,000 mPa·s) at a high temperature (50 to 100°C) and a low viscosity (100 to 580 mPa·s) at a low temperature (0 to 25°C).

In one specific embodiment, the temperature-responsive hydrogel exhibits a high viscosity (1,000 to 2,500 mPa·s) at a high temperature (50 to 100°C) and a low viscosity (100 to 500 mPa·s) at a low temperature (0 to 25°C).

In one specific embodiment, the temperature-responsive hydrogel exhibits a high viscosity (1,000 to 5,000 mPa·s) at a high temperature (50 to 100°C) and a low viscosity (100 to 500 mPa·s) at a low temperature (0 to 25°C).

In one specific embodiment, the temperature-responsive hydrogel exhibits a high viscosity (3,000 to 10,000 mPa·s) at a high temperature (50 to 100°C) and a low viscosity (500 to 2,980 mPa·s) at a low temperature (0 to 25°C).

In one specific embodiment, the temperature-responsive hydrogel exhibits a high viscosity (3,000 to 10,000 mPa·s) at a high temperature (50 to 100°C) and a low viscosity (1,000 to 2,980 mPa·s) at a low temperature (0 to 25°C).

The expression "a high viscosity at a high temperature" refers to the case where the hydrogel exhibits a viscosity within a high-viscosity range at any temperature within a high-temperature range, and the expression "a low viscosity at a low temperature" refers to the case where the hydrogel exhibits a viscosity within a low-viscosity range at any temperature within a low-temperature range. Preferably, the temperature-responsive hydrogel exhibits a viscosity within a high-viscosity range at all temperatures within a high-temperature range and a viscosity within a low-viscosity range at all temperatures within a low-temperature range.

The temperature-responsive hydrogel of the present invention is characterized by exhibiting a reversible (repeatable or reproducible) response (change in viscosity) in association with a change in temperature. Even when the temperature-responsive hydrogel of the present invention undergoes repeated cycles of temperature change (e.g., low temperature → high temperature → low temperature → high temperature), the temperature-responsive hydrogel can maintain a temperature response such that the viscosity increases with an increase in temperature and the viscosity decreases with a decrease in the increased temperature, or the viscosity decreases with a decrease in temperature and the viscosity increases with an increase in the decreased temperature.

For example, the temperature-responsive hydrogel of the present invention preferably exhibits a temperature response such that when the temperature of the hydrogel is increased from a low temperature to a high temperature within a temperature range wherein the hydrogel exhibits its gel characteristics, the viscosity of the hydrogel doubles or more, and when the temperature of the hydrogel is decreased from the high temperature to the low temperature (i.e., original temperature), the hydrogel exhibits substantially the same viscosity as that at the same temperature as that before the temperature change. The expression "the same viscosity at the same temperature" refers to the case where the ratio of the viscosity of the temperature-responsive hydrogel after the temperature change to the viscosity of the temperature-responsive hydrogel before the temperature change is, for example, 0.5 to 1.5, for example, 0.8 to 1.2, for example, 0.9 to 1.1.

Specifically, as described in the Examples below, the temperature-responsive hydrogel of the present invention exhibits substantially the same viscosity at the same temperature even when the hydrogel undergoes repeated cycles of temperature change (e.g., 4°C → 25°C → 70°C → 4°C). The temperature-responsive hydrogel exhibits such repeatability (reproducibility) at least once, for example, twice, for example, three times, for example, four times, for example, five times, for example, six times, for example, 10 times, for example, 50 times, for example, 100 times, for example, 101 times or more.

### [Preparation of Temperature-Responsive Hydrogel]

No particular limitation is imposed on the method for preparing the temperature-responsive hydrogel of the present invention, so long as the method involves mixing of the components of the hydrogel; i.e., the polyalkylene glycol (A), the silicate salt (B), and water. For example, the method involves previous preparation of an aqueous solution of the polyalkylene glycol (A) and an aqueous dispersion of the silicate salt (B), and subsequent mixing of the two aqueous liquids. The method may optionally involve addition of the dispersant (C) and/or the water-soluble organic solvent (D) before, during, or after mixing of the aqueous solution and the aqueous dispersion. In particular, addition of the dispersant (C) and/or the water-soluble organic solvent (D) to either or both of the aqueous solution of the polyalkylene glycol (A) and the aqueous dispersion of the silicate salt (B) before mixing of the aqueous solution and the aqueous dispersion facilitates production of a temperature-responsive gel in which the components are uniformly dispersed.

In the present invention, the aqueous solution and the aqueous dispersion are preferably prepared so that the mixture of the aqueous solution and the aqueous dispersion contains the components in small amounts, in order to increase the fluidity of the temperature-responsive hydrogel during molding of the hydrogel.

The method for mixing of the components in the aqueous solution or the aqueous dispersion can involve mechanical or manual stirring, or otherwise ultrasonic treatment. Preferably, mechanical stirring is used. The mechanical stirring may involve the use of, for example, a magnetic stirrer, a blade-type stirrer, a rotation-revolution mixer, a disper, a homogenizer, a shaker, a vortex mixer, a ball mill, a kneader, a line mixer, or an ultrasonic oscillator. Of these, a magnetic stirrer, a blade-type stirrer, a rotation-revolution mixer, or a line mixer is preferably used for mixing.

The mixing of the above-prepared liquids (the aqueous solution and the aqueous dispersion) is performed at a temperature ranging from the freezing point to the boiling point of the aqueous solution or the aqueous dispersion, preferably -5°C to 100°C, more preferably 0°C to 70°C.

### [Molding of Temperature-Responsive Hydrogel]

The aqueous solution of the polyalkylene glycol (A) can be mixed with the aqueous dispersion of the silicate salt (B), and the mixture can be poured into a container, such as a petri dish, a vat, or a tray, to thereby form the hydrogel into any shape (e.g., a sheet-like shape). Also, the hydrogel can be formed into a thin film by application or spin coating.

The aforementioned gelation is performed at a temperature of -5°C to 200°C, preferably 0°C to 150°C, more preferably 10°C to 90°C.

### Examples

The present invention will next be described in detail by way of examples, but the present invention is not limited to the following examples.

### [Example 1]

A transparent aqueous dispersion containing 4.0% by mass a water-swellable layered clay mineral (LAPONITE XLG: synthetic hectorite, manufactured by Wilbur-Ellis) as a silicate salt was prepared, and an aqueous PEG solution containing 0.2% by mass polyethylene glycol 500,000 (PEG) (molecular weight: about 500,000, manufactured by Wako Pure Chemical Industries, Ltd.) as a polyalkylene glycol was prepared. Subsequently, while the aqueous LAPONITE XLG dispersion was stirred, an equal amount of the aqueous PEG solution was added dropwise to the dispersion. After completion of the dropwise addition, the resultant mixture was further stirred for one hour, to thereby prepare an aqueous LAPONITE XLG/PEG dispersion having a LAPONITE XLG concentration of 2.0% by mass and a PEG concentration of 0.1% by mass.

### [Example 2]

An aqueous LAPONITE XLG/PEG dispersion was prepared in the same manner as in Example 1, except that the aqueous PEG solution was replaced with an aqueous PEG solution containing 0.4% by mass PEG. The aqueous LAPONITE XLG/PEG dispersion was found to have a LAPONITE XLG concentration of 2.0% by mass and a PEG concentration of 0.2% by mass.

### [Example 3]

An aqueous LAPONITE XLG/PEG dispersion was prepared in the same manner as in Example 1, except that the aqueous PEG solution was replaced with an aqueous PEG solution containing 0.8% by mass polyethylene glycol (PEG). The aqueous LAPONITE XLG/PEG dispersion was found to have a LAPONITE XLG concentration of 2.0% by mass and a PEG concentration of 0.4% by mass.

### [Comparative Example 1]

An aqueous LAPONITE XLG/PEG dispersion was prepared in the same manner as in Example 1, except that the aqueous PEG solution was replaced with water containing no polyethylene glycol (PEG). The aqueous LAPONITE XLG/PEG dispersion was found to have a LAPONITE XLG concentration of 2.0% by mass and a PEG concentration of 0% by mass.

### [Referential Example 1]

An aqueous LAPONITE XLG/PEG dispersion was prepared in the same manner as in Example 1, except that the aqueous PEG solution was replaced with an aqueous PEG solution containing 2.0% by mass polyethylene glycol (PEG). The aqueous LAPONITE XLG/PEG dispersion was found to have a LAPONITE XLG concentration of 2.0% by mass and a PEG concentration of 1.0% by mass.

### <Measurement of Viscosity>

The viscosity of each of the aqueous LAPONITE XLG/PEG dispersions prepared in Examples 1 to 3, Comparative Example 1, and Referential Example 1 was measured at a temperature of 25°C with a tuning-fork vibration viscometer (SV-100) manufactured by A&D Company, Limited. The viscosity was defined as the average of values obtained by five-minute measurement. The results are shown in Table 1.

**Table 1**

| | Composition (% by mass) | | Viscosity |
|---|---|---|---|
| | LAPONITE XLG | PEG | (mPa·s) |
| Example 1 | 2.0 | 0.1 | 460 |
| Example 2 | 2.0 | 0.2 | 550 |
| Example 3 | 2.0 | 0.4 | 380 |
| Comparative Example 1 | 2.0 | 0 | 3 |
| Referential Example 1 | 2.0 | 1.0 | 11 |

As shown in Table 1, the aqueous LAPONITE XLG/PEG dispersions of Examples 1 to 3 (PEG concentrations: 0.1, 0.2, and 0.4% by mass) were in the form of a hydrogel. In particular, the aqueous LAPONITE XLG/PEG dispersion of Example 2 (PEG concentration: 0.2% by mass) exhibited the highest viscosity of all aqueous LAPONITE XLG/PEG dispersions of the Examples. In contrast, the dispersion of Comparative Example 1 (containing no PEG) and the dispersion of Referential Example 1 (PEG concentration: 1% by mass) exhibited a very low viscosity and were in the form of a sol (aqueous solution).

### <Change in Viscosity with Temperature>

The viscosity of the aqueous LAPONITE XLG/PEG dispersion prepared in Example 2 was measured with a tuning-fork vibration viscometer (SV-100) manufactured by A&D Company, Limited under repeated cycles of measurement temperature change (4°C → 25°C → 70°C → 4°C). The viscosity was defined as the average of values obtained by five-minute measurement. The cycle of measurement temperature change was repeated six times.

Table 2 shows the average of viscosities measured at each temperature in six repeated cycles of temperature change. In all the six repeated cycles of temperature change, the aqueous LAPONITE XLG/PEG dispersion exhibited a high viscosity at a high temperature and a low viscosity at a low temperature. As shown in Table 2, the aqueous LAPONITE XLG/PEG dispersion exhibited a temperature response such that the viscosity at a temperature of 70°C was about six times the viscosity at a temperature of 4°C.

**Table 2**

| | Composition (% by mass) | | Viscosity (mPa·s) | | |
|---|---|---|---|---|---|
| | LAPONITE XLG | PEG | 4°C | 25°C | 70°C |
| Example 2 | 2.0 | 0.2 | 204 | 460 | 1200 |

### [Example 4]

An aqueous LAPONITE XLG/PEG dispersion was prepared in the same manner as in Example 1, except that the aqueous LAPONITE XLG dispersion was replaced with a transparent aqueous dispersion containing 6.0% by mass LAPONITE XLG. The aqueous LAPONITE XLG/PEG dispersion was found to have a LAPONITE XLG concentration of 3.0% by mass and a PEG concentration of 0.1% by mass.

### [Example 5]

An aqueous LAPONITE XLG/PEG dispersion was prepared in the same manner as in Example 1, except that the aqueous LAPONITE XLG dispersion was replaced with a transparent aqueous dispersion containing 6.0% by mass LAPONITE XLG, and the aqueous PEG solution was replaced with an aqueous PEG solution containing 0.4% by mass PEG. The aqueous LAPONITE XLG/PEG dispersion was found to have a LAPONITE XLG concentration of 3.0% by mass and a PEG concentration of 0.2% by mass.

### [Example 6]

An aqueous LAPONITE XLG/PEG dispersion was prepared in the same manner as in Example 1, except that the aqueous LAPONITE XLG dispersion was replaced with a transparent aqueous dispersion containing 6.0% by mass LAPONITE XLG, and the aqueous PEG solution was replaced with an aqueous PEG solution containing 1.2% by mass polyethylene glycol (PEG). The aqueous LAPONITE XLG/PEG dispersion was found to have a LAPONITE XLG concentration of 3.0% by mass and a PEG concentration of 0.6% by mass.

### [Comparative Example 3]

An aqueous LAPONITE XLG/PEG dispersion was prepared in the same manner as in Example 1, except that the aqueous LAPONITE XLG dispersion was replaced with a transparent aqueous dispersion containing 6.0% by mass LAPONITE XLG, and the aqueous PEG solution was replaced with an aqueous PEG solution containing 4.0% by mass polyethylene glycol (PEG). The aqueous LAPONITE XLG/PEG dispersion was found to have a LAPONITE XLG concentration of 3.0% by mass and a PEG concentration of 2.0% by mass.

### <Measurement of Viscosity>

The viscosity of each of the aqueous LAPONITE XLG/PEG dispersions prepared in Examples 4 to 6 and Comparative Example 3 was measured at a temperature of 25°C with a tuning-fork vibration viscometer (SV-100) manufactured by A&D Company, Limited. The viscosity was defined as the average of values obtained by five-minute measurement. The results are shown in Table 3.

**Table 3**

| | Composition (% by mass) | | Viscosity |
|---|---|---|---|
| | LAPONITE XLG | PEG | (mPa·s) |
| Example 4 | 3.0 | 0.1 | 2280 |
| Example 5 | 3.0 | 0.2 | 2730 |
| Example 6 | 3.0 | 0.6 | 1890 |
| Comparative Example 3 | 3.0 | 2.0 | 167 |

The aqueous LAPONITE XLG/PEG dispersions prepared in Examples 4 to 6 were in the form of a hydrogel and exhibited a high viscosity of about 2,000 to 3,000 mPa·s. In contrast, the aqueous LAPONITE XLG/PEG dispersion prepared in Comparative Example 3 exhibited a low viscosity of 167 mPa·s despite a high PEG concentration.

The aqueous LAPONITE XLG/PEG dispersion prepared in Example 6 was evaluated for change in viscosity with temperature in the same manner as in Example 2. Consequently, the viscosity was 5,500 mPa·s at a high temperature (70°C) and 1,890 mPa·s at a low temperature (25°C). Thus, the aqueous LAPONITE XLG/PEG dispersion prepared in Example 6 also exhibited a temperature response such that the viscosity at a high temperature was higher than the viscosity at a low temperature.

### [Comparative Example 4]

An aqueous solution containing 0.2% by mass polyethylene glycol 500,000 (PEG) (molecular weight: about 500,000) only was prepared.

### [Comparative Example 5]

An aqueous dispersion containing 2% by mass Laponite XLG only was prepared.

### < Test for Temperature Dependence of Viscosity>

The viscosities of the aqueous solution prepared in Comparative Example 4 and the aqueous dispersion prepared in Comparative Example 5 were measured under repeated cycles of temperature change (4°C → 25°C → 70°C → 4°C). Consequently, the aqueous solution and the aqueous dispersion exhibited a common temperature dependence of viscosity; i.e., a low viscosity at a high temperature (70°C) (12.9 mPa·s (Comparative Example 4) and 3.5 mPa·s (Comparative Example 5)) and a high viscosity at a low temperature (4°C) (15.7 mPa·s (Comparative Example 4) and 4.6 mPa·s (Comparative Example 5)).

**Table 4**

| | Composition (% by mass) | | Viscosity (mPa·s) | | |
|---|---|---|---|---|---|
| | LAPONITE XLG | PEG | 4°C | 25°C | 70°C |
| Example 2* | 2.0 | 0.2 | 204 | 460 | 1200 |
| Comparative Example 4 | 0 | 0.2 | 15.7 | - | 12.9 |
| Comparative Example 5 | 2.0 | 0 | 4.6 | - | 3.5 |

| | | | | | |
|---|---|---|---|---|---|
| *: Redescribed | | | | | |

### INDUSTRIAL APPLICABILITY

The hydrogel of the present invention can be prepared only by mixing of inexpensive raw materials without use of a chemical reaction, such as radical polymerization. The hydrogel can be used as a temperature-responsive hydrogel by taking advantage of its characteristics.

## Claims

1. A temperature-responsive hydrogel comprising a polyalkylene glycol (A) and a silicate salt (B), wherein the concentration by mass of the polyalkylene glycol (A) is 0.01 to 1.5% by mass in the hydrogel.

2. The temperature-responsive hydrogel according to claim 1, wherein the temperature-responsive hydrogel further comprises a dispersant (C) for the silicate salt.

3. The temperature-responsive hydrogel according to claim 1 or 2, wherein the polyalkylene glycol (A) is polyethylene glycol or polypropylene glycol having a weight average molecular weight of 20,000 to 10,000,000.

4. The temperature-responsive hydrogel according to any one of claims 1 to 3, wherein the concentration by mass of the silicate salt (B) is 0.5 to 10% by mass in the hydrogel.

5. The temperature-responsive hydrogel according to any one of claims 1 to 4, wherein the silicate salt (B) is water-swellable silicate salt particles.

6. The temperature-responsive hydrogel according to claim 5, wherein the water-swellable silicate salt particles are particles of a water-swellable silicate salt selected from the group consisting of smectite, bentonite, vermiculite, and mica.

7. The temperature-responsive hydrogel according to any one of claims 1 to 6, wherein the mass ratio R of the polyalkylene glycol (A) to the silicate salt (B) (R = [the mass of A]/[the mass of B]) is 0.01 to 0.5.

8. The temperature-responsive hydrogel according to any one of claims 2 to 7, wherein the dispersant (C) is at least one dispersant selected from the group consisting of sodium pyrophosphate, citric acid, and polyphosphoric acid.

9. The temperature-responsive hydrogel according to any one of claims 1 to 8, wherein the viscosity of the hydrogel at a temperature of 50 to 100°C is twice or more the viscosity of the hydrogel at a temperature of 0 to 25°C.

10. The temperature-responsive hydrogel according to any one of claims 1 to 8, wherein the hydrogel exhibits a viscosity of 600 to 10,000 mPa·s at a temperature of 50 to 100°C and a viscosity of 100 to 580 mPa·s at a temperature of 0 to 25°C.

11. The temperature-responsive hydrogel according to any one of claims 1 to 8, wherein the hydrogel exhibits a viscosity of 3,000 to 10,000 mPa·s at a temperature of 50 to 100°C and a viscosity of 500 to 2,980 mPa·s at a temperature of 0 to 25°C.

12. A method for producing a temperature-responsive hydrogel, the method comprising a step of mixing an aqueous solution containing a polyalkylene glycol (A) at a concentration by mass of 0.02 to 3.0% by mass with an aqueous dispersion containing a silicate salt (B).

13. The method for producing a temperature-responsive hydrogel according to claim 11, wherein one or more water-soluble organic solvents (D) are contained in either or both of the aqueous solution and the aqueous dispersion before mixing of the aqueous solution and the aqueous dispersion.

14. The temperature-responsive hydrogel according to any one of claims 9 to 11, wherein the temperature-responsive hydrogel exhibits at least one reproducible change in viscosity of the hydrogel with respect to a change in temperature.
